# EUROPEAN PATENT SPECIFICATION

(11) **EP 2 410 942 B1**
(45) Date of publication and mention of the grant of the patent: **11.12.2019**
(21) Application number: 09842426.0
(22) Date of filing: 27.03.2009
(51) Int. Cl.: A61K 6/00, A61K 6/083, A61K 6/09

(54) **CURABLE ZIRCONIA ADHESIVE COMPOSITIONS FOR DENTAL RESTORATIONS**
HÄRTBARE ZIRKONIUMDIOXID-KLEBEZUSAMMENSETZUNGEN FÜR ZAHNRESTAURATIONEN
COMPOSITIONS ADHÉSIVES DURCISSABLES À BASE DE ZIRCONE POUR RESTAURATIONS DENTAIRES

(43) Date of publication of application: 01.02.2012
(73) Proprietor: Bisco, Inc., Schaumburg, IL 60193 (US)
(72) Inventor: SUH, Byoung, Oak Brook,IL 60523 (US); CHEN, Liang, Schaumburg, IL 60193 (US)
(74) Representative: Elsy, David
(86) International application number: PCT/US2009/038575
(87) International publication number: WO 2010/110798

(56) References cited:
- US-A- 6 147 137
- US-A- 6 147 137
- US-A1- 2004 254 261
- US-A1- 2005 252 414
- US-A1- 2008 293 846
- US-A1- 2008 293 846
- US-B1- 6 512 068
- US-B2- 6 730 715
- US-B2- 6 730 715
- US-B2- 6 750 268

## Description

### BACKGROUND

Aesthetic and biocompatibility considerations have greatly increased the demand for metal-free dental restorations in clinical dentistry, leading to great demand to replace the once-common metal or metal-backed ceramic fillings, crowns, veneers, bridges, posts, and other dental prosthetics and restorations with ceramic (also known as full-porcelain) versions of those restorations. However, traditional glass-ceramic and aluminum oxide ceramic restorations display a brittleness, high propensity for crack propagation, low tensile strength, and poor wear resistance that limits their use or longevity in many applications for dental restorations.

Zirconia-based technologies, such as zirconium-oxide materials, have greatly overcome the poor performance properties of traditional ceramic restorations with their high strength and comparatively higher fracture toughness, and may be used in endodontic posts, implants, and implant abutments, orthodontic brackets, cores for crowns, and fixed partial denture prosthesis frameworks, and other dental restorations. Further, zirconia provides the metal-free, aesthetic characteristics requested by patients, and its hard and dense surface is ideal for resisting wear damage making zirconia an attractive material for single tooth dental restorations.

However, while zirconia dental restoration materials (including those sold under the LAVA, CERCON, and PROCERA trademarks) show a marked improvement in wear and strength properties in dental applications over traditional ceramics, these materials have proven to be challenging to adhere to dentin (whether etched or unetched), enamel, resins, and other materials using traditional dental materials and techniques. The questions of how to prepare the internal surfaces of restorations and restoration cites, as well as what proper adhesive protocols will result in clinically optimal results are current challenges of zirconia bonding because the clinically established protocol of etching (i.e., with hydrogen fluoride) and silanation, effective for other glass ceramic materials, does not yield sufficient strength when applied in zirconia bonding. Thus the acid-resistant, silica free surface of zirconia creates difficulty in establishing a strong and stable bond between the zirconia internal surface and tooth structure.

U.S. Patent Publication No. 2004/02544261 to Kojima et al. discloses dental compositions including a polymerizable component with an acidic group and an aluminum oxide powder as a preservative, and may include fillers such as zirconia. US Patent Publication No. 2005/0252414 to Craig et al. discloses dental compositions including a polymerizable component comprising an ethylenically unsaturated compound with acid functionality and a nanofiller that may include zirconia. U.S. Patent Publication No. 2008/0293846 to Craig et al. discloses dental compositions comprising a basic filler with an acidic component, such as a carboxylic acid attached to the surface of the filler. The basic filler comprises a metal oxide particle, such as zirconia. U.S. Patent No. 6,730,715 to Jia discloses dental composite materials comprising an ethylenically unsaturated monomer or oligomer having one or multiple carboxylic acid groups. The composition may further comprise a filler, such as zirconia. U.S. Patent No. 6,147,137 to Jia discloses dental compositions comprising a polymerizable acidic component, such as a monomer having a polymerizable acrylate or methacrylate group and an aromatic carboxylic acid group. The composition may further comprise a filler, such as zirconium oxide.

Previous attempts to improve the adhesion between resin materials and zirconia include U.S. Patent 6,939,901 to Nakatsuka et al. This system utilizes a two-part adhesive system comprising a first polymerizable monomer containing an acidic group and a second polymerizable monomer having a general formula of 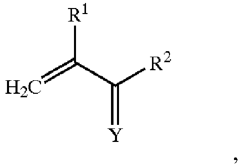 with R¹ being hydrogen or a methyl group; R² being a halogen; hydroxyl group, mercapto group, or -O-R³-OH group; R³ being an alkene group having 6 to 25 carbons; and Y being oxygen or sulfur. Similarly, U.S. Patent 6, 512,068 to Nakatsuka utilizes an adhesive system comprising a water-insoluble acid monomer having an alkene group of 8-25 carbons atoms, an alkyl group having 8-25 carbon atoms, an aromatic group, and an acid group selected from phosphoric acid, thiophosphoric acid, carboxylic acid, sulfonic acid, or another similar acid group, and a polymerizable unsaturated group selected from an acryloyl group, a methacryloyl group, a vinyl group, a styrene group; wherein the water-insoluble acid is present as a salt by combining the water-insoluble acid with a base in water to create a composition with a pH of 1.0 to 6.0. However, tests of commercial embodiments of these systems reveal bonding strengths between zirconia materials and other dental materials that would preferably be higher in clinical applications. Therefore an improved system for bonding, including greater bond strength and durability of bond between both resin materials and zirconia, as well as bonding between etched and un-etched dentin and resin materials in clinical applications would be appreciated in the art.

### SUMMARY

The invention is defined in the appended claims and includes a dental restoration system comprising a zirconia-based dental appliance; and an adhesive composition operable to bond materials to the zirconia-based dental applicance; the adhesive composition comprising a first acidic monomer having an ethylenically unsaturated polymerizable group of the general formula CH₂=CX-C(O)-R wherein X is hydrogen, methyl or a lower alkyl group, and wherein R comprises a phosphoric acid group or phosphonic acid group having the general formula -OP(O)(OH)₂, - OP(O)(OH), -C-P(O)(OH)₂ or -C-P(O)(OH); a second acidic monomer having an ethylenically unsaturated polymerizable group of the general formula CH₂=CX-C(O)-R₁ wherein X is hydrogen, methyl or a lower alkyl group, and wherein R₁ comprises a carboxylic acid group; wherein the first acidic monomer comprises 1% to 5% of the dental adhesive composition by weight; and wherein the second acidic monomer comprises 2% to 10% of the dental adhesive system by weight.

According to at least one embodiment, the abovementioned system optionally utilizes the first acidic monomer selected from the group consisting of CH₂=C(CH₃)COO(CH₂)ₙOP(O)(OH)₂ and CH₂=C(CH₃)COO(CH₂)ₙP(O)(OH)₂, wherein n is an integer from 2 to 20.

According to at least one embodiment, the abovementioned system optionally utilizes the second acidic monomer selected from the group consisting of tetrahydrofurfuryl cyclohexene dimethacrylate; the reaction product of 3,3'4,4'-diphenylsulfone tetracarboxylic dianhydride and 2-hydroxyethyl methacrylate; or biphenyl dimethacrylate. Further optionally, the dental adhesive composition comprises at least one comonomer selected from the group consisting of 2-hydroxyethyl methacrylate, bisphenol A diglycidyl methacrylate, triethylene glycol dimethacrylate, 1,6-hexanediol dimethacrylate, ethoxylated bisphenol A diglycidyl methacrylate, and urethane dimethacrylate. Further optionally, the dental adhesive composition comprises an initiator.

In at least one optional embodiment, the dental adhesive composition utilizes an initiator comprising at least one photosensitive ketone and at least one tertiary amine. Further optionally, neither of the acidic monomers of the dental adhesive composition contain a halogen group.

According to at least one aspect, the dental adhesive composition comprises a ratio of the first acidic monomer to the second acidic monomer that is between 1:10 and 3:1, respectively. Further optionally, the dental adhesive composition comprises a ratio of the first acidic monomer to the second acidic monomer that is between 1:4 and 2:1, respectively. Further optionally, the dental adhesive composition comprises a ratio of the first acidic monomer to the second acidic monomer that is between 1:3.5 and 1:1, respectively.

Other embodiments disclosed herein relate to a dental adhesive composition comprising a first acidic monomer having an ethylenically unsaturated polymerizable group of the general formula CH₂=CX-C(O)-R wherein X is hydrogen, methyl or a lower alkyl group, and wherein R is a phosphoric acid group or phosphonic acid group; a second acidic monomer having an ethylenically unsaturated polymerizable group of the general formula CH₂=C_{X}-C(O)-R₁ wherein X is hydrogen, methyl or a lower alkyl group, and wherein R₁ comprises a carboxylic acid group; at least one comonomer selected from the group consisting of 2-hydroxyethyl methacrylate, bisphenol A diglycidyl methacrylate, triethylene glycol dimethacrylate, 1,6-hexanediol dimethacrylate, ethoxylated bisphenol A diglycidyl methacrylate, and urethane dimethacrylate; an initiator comprising at least one photosensitive ketone and at least one tertiary amine. As disclosed herein, a ratio of the first acidic monomer to the second acidic monomer may be selected from a range between: 1:10 and 3:1, respectively; 1:4 and 2:1, respectively; and 1:3.5 and 1:1, respectively.

The first acidic monomer comprises 1% to 5% of the dental adhesive composition by weight. The second acidic monomer comprises 2% to 10% of the dental adhesive composition by weight. In at least one optional embodiment, the comonomer comprises 4% to 36% of the dental adhesive composition by weight.

According to at least one optional embodiment, the at least one comonomer comprises two comonomers selected from the group consisting of 2-hydroxyethyl methacrylate, bisphenol A diglycidyl methacrylate, triethylene glycol dimethacrylate, 1,6-hexanediol dimethacrylate, ethoxylated bisphenol A diglycidyl methacrylate, and urethane dimethacrylate. Further optionally, the initiator comprises 0.05% to 3% of the dental adhesive composition by weight. In yet at least one other optional embodiment, the initiator comprises at least one photosensitive ketone and at least one tertiary amine, and optionally comprises at least one solvent.

According to certain embodiments, the dental adhesive composition displays an initial bond strength to zirconia that is greater than 10 MPa as measured with the #5 gel cap method when polymerized. Further optionally, the adhesive composition displays an initial bond strength to zirconia that is at least 14 MPa as measured with the #5 gel cap method when polymerized.

The present invention also relates to a method of bonding a zirconia-based dental appliance to a surface, comprising providing a first acidic monomer having an ethylenically unsaturated polymerizable group of the general formula CH₂=CX-C(O)-R wherein X is hydrogen, methyl or a lower alkyl group, and wherein R is a phosphoric acid group or phosphonic acid group having the general formula - OP(O)(OH)₂, -OP(O)(OH), -C-P(O)(OH)₂ or -C-P(O)(OH), the first acidic monomer comprising 1% to 5% of the provided materials by weight; providing a second acidic monomer having an ethylenically unsaturated polymerizable group of the general formula CH₂=C_{X}-C(O)-R₁ wherein X is hydrogen, methyl or a lower alkyl group, and wherein R₁ comprises a carboxylic acid group, the second acidic monomer comprising 2% to 10% of the provided materials by weight; providing at least one comonomer selected from the group consisting of 2-hydroxyethyl methacrylate, bisphenol A diglycidyl methacrylate, triethylene glycol dimethacrylate, 1,6-hexanediol dimethacrylate, ethoxylated bisphenol A diglycidyl methacrylate, and urethane dimethacrylate, the comonomer comprising 4% to 36% of the provided materials by weight; providing an initiator comprising at least one photosensitive ketone and at least one tertiary amine, the initiator comprising 0.05% to 3% of the provided materials by weight; combining all provided materials and applying those materials to a zirconia-based dental appliance; and polymerizing the combined materials applied to the zirconia-based dental appliance such that the initial bond between the polymerized materials and the zirconia is at least 10 MPa as measured with the #5 gel cap method.

The method utilizes the provided materials in the following percentages: the first acidic monomer comprises 1% to 5% of the provided materials by weight; the second acidic monomer comprises 2% to 10% of the of the provided materials by weight; the comonomer comprises 4% to 36% of the provided materials by weight; the initiator comprises 0.05% to 3% of the dental adhesive composition by weight; and a solvent comprises 40% to 90% of the provided materials by weight.

In yet at least one additional optional embodiment, none of the provided monomers comprise a halogen group. Further optionally, the initial bond between the polymerized materials and the zirconia is at least 14 MPa as measured with the #5 gel cap method. As an additional option, the provided materials are polymerized via light curing. Finally, as an additional option, the bond between the polymerized materials and the zirconia is at least 10 MPa as measured with the #5 gel cap method when exposed to water at 100° C for 72 hours.

### DESCRIPTION

### A. Dental Restoration System

A dental restoration system comprising a zirconia-based dental appliance and an adhesive composition applied to the surface of the zirconia-based dental appliance is provided herein for creating or enhancing a strong and lasting bond between dental resins, etched and unetched dentin, cements, composites and other dental materials to zirconia ceramics as well as other dental substrates, such as dentin, metal, and other ceramics. The dental adhesive composition comprises a first monomer and a second monomer, wherein each monomer is acidic.

The dental adhesive composition comprises a first polymerizable acidic monomer and a second polymerizable acidic monomer, wherein the first polymerizable acidic monomer comprises an ethylenically unsaturated polymerizable group or moiety of the general formula CH₂=C_{X}-C(O)-R wherein X is hydrogen, methyl or a lower alkyl group, and R comprises a phosphoric or phosphonic acid group of general formula -OP(O)(OH)₂, -OP(O)(OH), -C-P(O)(OH)₂ or -C-P(O)(OH), wherein the first acidic monomer comprises 1% to 5% of the adhesive composition by weight. For exemplary purposes, the phosphoric or phosphonic acid group is methacryloyloxyalkyl dihydrogenphosphates CH₂=C(CH₃)COO(CH₂)ₙOP(O)(OH)₂ or CH₂=C(CH₃)COO(CH₂)ₙP(O)(OH)₂ wherein n is an integer from 2 to 20. Additionally, the second polymerizable acidic monomer comprises an ethylenically unsaturated polymerizable group or moiety of the general formula CH₂=C_{X}-C(O)-R₁ wherein X is hydrogen, methyl or a lower alkyl group, and R₁ comprises a carboxylic acid group of the general formula -C(O)OH, wherein the second acidic monomer comprises 2% to 10% of the adhesive composition be weight. For exemplary purposes, the carboxylic acidic group may be tetrahydrofurfuryl cyclohexene dimethacrylate ("TCDM", the reaction product of Epiclon B-4400 with HEMA, available from Dainippon Inc. and Chemicals Inc., Ft. Lee, N.J.); the reaction product of 3,3'4,4'-diphenylsulfone tetracarboxylic dianhydride and 2-HEMA (hereinafter "DSDM") or biphenyl dimethacrylate (hereinafter "BPDM", the reaction product of an aromatic dianhydride with an excess of 2-HEMA as described in U.S. Pat. No. 5,348,988). For convenience, the structures of each of these exemplary carboxylic acid groups are set forth below:

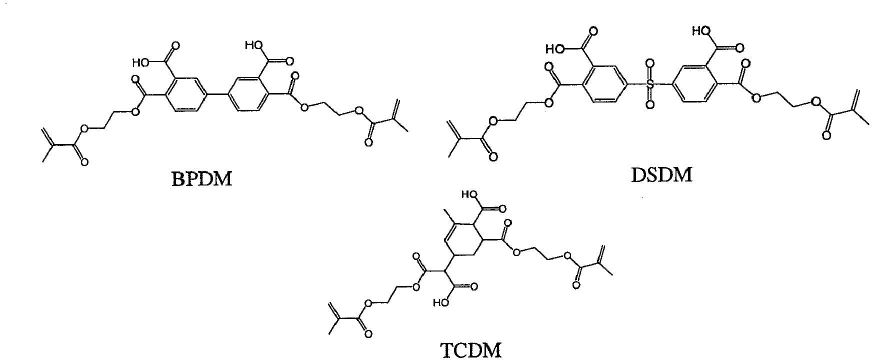

According to at least one embodiment, a dental adhesive composition for bonding a zirconia-based dental appliance may further comprise an additional monomer or monomers, hereinafter referred to as "comonomers", which may include monomethacrylates such as 2-hydroxyethyl methacrylate (HEMA) and dimethacrylates such as bisphenol A diglycidyl methacrylate (BisGMA), triethylene glycol dimethacrylate (TEGDMA), 1,6-hexanediol dimethacrylate (HDDMA), ethoxylated bisphenol A diglycidyl methacrylate (BisEMA), and urethane dimethacrylate (UDMA). Further, according to at least one embodiment, a dental adhesive composition may comprise one or more solvents, including water, ethanol, acetone, or other solvents utilized with dental monomers, or mixtures thereof. According to at least one embodiment, a dental adhesive composition further comprises an initiator operable to promote polymerization of the adhesive composition. According to at least one exemplary embodiment, the initiator is a photoinitiator operable to initiate polymerization of the dental adhesive composition when exposed to a selected wavelength (such as ultraviolet or portions of the visible spectrum of light). Additionally, as disclosed herein, a dental adhesive composition may further comprise one or more silane coupling agents, such as 3-methacryloxypropyl tris(trimethylsiloxy)silane ("MPTS").

In the event that a photoinitiated adhesive composition is desired, according to at least one embodiment, the initiator may comprise one or more photosensitive ketones and may optionally include a tertiary amine. Typical photosensitive ketones include benzophenone, acetophenone, thioxanthen-9-one, 9-fluorenone, anthraquinone, 4'-methoxyacetophenone, diethoxyacetophenone, biacetyl 2,3-pentadione, benzyl 4,4'-methoxybenzil, 4,4'-oxidibenzil, and camphroquinone (CQ). Typical tertiary amines include ethyl-4-dimethyl amino benzoate, ethyl-2-dimethyl amino benzoate ("EDMAB"), 4,4-bis(dimethylamino)benzophenone, n-methyldiethanolamine, and dimethylaminobenzaldehyde. According to one exemplary embodiment, a combination of CQ and EDMAB is utilized as an initiator. According to at least one exemplary embodiment, an initiator may comprise a photosensitive ketone in the dental adhesive composition in a concentration of 0.05 wt. % to 1 wt. %, and a tertiary amine with the concentration comprising 0.2 wt. % to 3 wt. % of the dental adhesive composition. According to yet another exemplary embodiment, an initiator may comprise a photosensitive ketone in the dental adhesive composition in a concentration of 0.2 wt. % to 0.4 wt. %, and a tertiary amine with the concentration comprising 0.6 wt. % to 1.5 wt. % of the dental adhesive composition.

Further, according to at least one embodiment of the present application a dental adhesive composition may be provided wherein a first part comprising an acidic monomer or monomers may be provided in a separate container from a second part comprising other comonomers and initiator systems, or wherein both the first part and the second part are supplied in a single container. In the event that the first part and the second part are supplied in separate containers, it will be appreciated that the first part and second part may be admixed in predetermined proportions prior to application to the substrates, such as zirconia or a prepared tooth surface.

According to certain exemplary embodiments, the dental adhesive composition comprises: a first acidic acid monomer having a phosphoric or phosphonic acid group, a second acidic acid monomer having a carboxylic acid group, a solvent, a comonomer, and an initiator, wherein: (1) the first acidic acid monomer comprises 1 wt. % to 5 wt %, 1.4 wt. % to 3 wt. %, of the dental adhesive composition; (2) the second acidic acid monomer having a carboxylic acid group comprising 2 wt. % to 0 wt. %, or 3 wt. % to 8 wt. %; (3) the comonomer comprises 4 wt % to 36 wt % 10 wt. % to 30 wt. %, or 20 wt. % to 30 wt. %; and (4) the solvent comprises 40 wt. % to 90 wt. %, or 55 wt. % to 80 wt. %; and (5) the initiator comprises 0.05 wt. % to 3 wt. %, 0.05 wt. % to 2 wt. %, or 0.05 wt. % to 1.5 wt. %.

In selecting the combination of first acidic monomer, second acidic monomer, and any comonomer, solvent, initiator, and/or silane coupling agent comprising a dental adhesive composition, one of ordinary skill in the art will appreciate that properties such as stability of the adhesive composition at room temperature, high initial bond strengths (10 to 20 MPa or higher with #5 gel-cap shear bond test method, and 40 MPa when utilizing the Ultradent shear bond test method) may be considerations in selecting the individual elements and their concentrations in the resulting dental adhesive composition. Further, those of ordinary skill in the art will appreciate that variants of the above combinations may be employed to produce a polymerizable dental adhesive composition or primer system operable to provide a strong bond between zirconia-based dental materials and dentin, dental resins, cements, metals, and/or other materials.

### B. Application of Dental Restoration System

It will be appreciated that a dental restoration system as described herein may be utilized to bond dental zirconia-based ceramic restorations with other dental substrates such as metal, dentin, and ceramics. For example, a dental adhesive composition as described herein may be applied as a layer to coat to a surface of a zirconia-based ceramic dental appliance, with the dental adhesive composition optionally being allowed to polymerize. Optionally, the polymerization may be initiated through light, heat, free radical initiation, or other methods known in the art.

Thereafter, a cement, composite, or other dental restorative resin, luting composite, or other component is applied over the polymerized or unpolymerized dental adhesive composition, thereby providing improved strength between the zirconia-based dental appliance and the cement, composite, or other dental restorative resin, luting composite, or other component. Thereafter, the zirconia-based dental appliance may be adhered to a prepared surface such as prepared dentin or enamel, metal, composite or other dental resin or other restorative materials by copolymerizing the dental adhesive composition and the cement, composite, or other dental restorative resin, luting composite, or other component.

It will be appreciated that by applying the layer of dental adhesive composition over the zirconia-based dental appliance surface, the restoration system may then be bonded to other dental substrates such as metal, dentin, ceramics with improved strength in the bond such that zirconia restorations may be applied in clinical applications where they would have previously failed. Further, in certain applications, the dental adhesive composition may be used to bond a zirconia-based dental appliance directly to dentin, enamel, metal, or other surfaces with a strong bond. Additionally, in all applications, the zirconia-based dental appliance surface may optionally be left unprepared, polished, or sandblasted prior to applying the dental adhesive composition over the surface. It will be appreciated by those skilled in the art that the dental compositions and the methods of the present invention have significant utility in various restorative applications. In determining the efficacy and clinical applicability of the dental restoration systems, a series of tests were performed utilizing different embodiments of the dental adhesive composition described herein. As set forth below, the standard shear bond strength method and Ultradent shear bond strength method of testing strength were utilized to compare varying formulas of dental adhesive compositions as described herein.

### C. Exemplary Embodiments

The compositions set forth in the following tables are a subset of those prepared according to certain embodiments of the present application, and each was evaluated using the standard shear bond strength method and Ultradent shear bond strength method as summarized below. To ensure consistency, each of the substrates were prepared as followed, and each exemplary embodiment was tested according to the following protocol.

### 1. Preparation of Substrates

In preparation of testing the dental restoration systems set forth below, all substrates were treated as follows:

### a. Zirconia Ceramics, Metals, and other Dental Ceramics

Zirconia, metals, other metal oxide ceramics, and other dental ceramics were abraded with wet 600 or 320 grit silicon carbide ("SiC") paper and lightly sandblasted (or as indicated) with aluminum oxide (50 µm) to create clean surface for bonding. The prepared substrate surface was washed by a typical 3-way dental syringe and air-dried.

### b. Dentin Substrates

Extracted human teeth were embedded in resin discs, abraded on the facial surface with a model trimmer, and subsequently abraded with wet 600 or 320 grit SiC paper to create a flat and smooth dentin substrate for bonding. Those prepared dentin surfaces noted as being etched were etched by Uni-Etch® brand etchant (available from Bisco, Inc., Schaumburg, IL) and rinsed, and those not noted as etched surfaces were simply rinsed and blotted dry. The dentin surface was blot dried with a sponge pellet to remove visible moisture

### 2. Bond Strength Testing

### a. Standard Shear Bond Strength Test Procedure ("SSBS", #5 Gel Cap Method).

In establishing standard shear bond strength ("SSBS") of the following exemplary embodiments of the dental adhesive composition, one or two coats of the exemplary formulation of the adhesive composition indicated were applied to the prepared substrate's surface by a micro-brush. The amount of adhesive composition applied was sufficient to cover the surface of the substrate. Thereafter, the applied adhesive composition was then air dried, and light cured ("LC") for 20 second at 500 mW/cm². A #5 gel cap (bonding area 0.1684 cm²) was filled with a dental cement, in this case, Choice 2™ cement was utilized (available from Bisco Inc.) unless otherwise indicated. Thereafter, the cement or composite was placed on the prepared substrate surface. Any excess cement was removed with a micro-brush and light cured from 2 sides for 40 s (for LC specimens) or as indicated for self-cure (left for 10 min @ 37° C oven). The samples were stored in deionized (DI) water at 37° C for the indicated amount of time before being broken using Instron (Model 4466) with crosshead speed of 5 mm/min. Shear bond strength (SBS) was calculated in MPa by dividing the peak load by bonding area. The mean and standard deviations were calculated for several replications (n is as indicated) for each test.

### b. Ultradent Method-Shear Bond Strength Test Procedure ("USBS")

In establishing Ultradent shear bond strength ("USBS") of the following exemplary embodiments of the dental restoration system, one or two coats of the dental adhesive composition were applied to the prepared substrate's surface by a micro-brush in an amount sufficient to cover the surface. Thereafter, the applied adhesive composition was then air dried, and light cured ("LC") for 20 seconds at 500 mW/cm². The indicated cement or composite was thereafter applied to the coated substrate, and the sample was cured according to the manufacturer's instructions. Thereafter, the sample was tested according to the use of the Ultradent shear bond test by using an Ultradent jig as described generally in the article: Pashley et al., Dent. Mater. 11 : 117-125 (1995). Each sample was stored in 37° C water for the indicated amount of time before being broken using Instron (Model 4466) with crosshead speed of 1 mm/min. Shear bond strength (SBS) was calculated in MPa by dividing the peak load by bonding area. Based on the diameter, 2.3798 mm, 1 1b. equals 1 MPa. The mean and standard deviations were calculated for several replications (n is indicated in each table) for each test, with the results set forth below.

### EXAMPLES

### I. Dental Adhesive Composition on Zirconia Surface

According to at least one exemplary embodiment, a dental adhesive composition was prepared utilizing the components and concentrations as set forth in Table 1. As will be seen, for the following exemplary embodiments, absolute ethyl alcohol ("EtOH") was used as the solvent unless otherwise noted; 10-methacryloyloxydecyl dihydrogenphosphates CH₂=C(CH₃)COO(CH₂)₁₀OP(O)(OH)₂ ("MDP") was used as a first acidic acid monomer, DSDM was used as a second acidic acid monomer, the specified methacrylates of BisGMA and HEMA were provided as comonomers as provided in Table 1, with the resultant standard shear bond strength results listed. Each noted adhesive composition was mixed in a closed container by shaking the components until all monomers dissolved (approximately four to eight hours). In each of the shear bond strength tests performed in Table 1, Choice 2™ cement was utilized (available from Bisco Inc.) as the cement.

**TABLE 1**

| SBS on Zirconia using Dental Adhesive Compositions (SSBS, in MPa) | | | | | |
|---|---|---|---|---|---|
| MDP wt. % | DSDM wt. % | EtOH wt. % | Methacrylate wt. % | Initiator wt. % | SSBS in MPa (SD) (#5 gel-cap) |
| 1 | 10 | 57.75 | BisGMA 20, HEMA 10 | CQ 0.25, EDMAB 1 | 16.47 (0.89), 2 hrs in water, n=8 |
| 3 | 10 | 55.75 | BisGMA 20, HEMA 10 | CQ 0.25, EDMAB 1 | 16.63 (2.48), 24 hrs in water, n=14 |
| 3 | 10 | 55.75 | BisGMA 20, HEMA 10 | CQ 0.25, EDMAB 1 | 15.69 (2.05), 2 hrs in water, n=10 |
| 6 | 10 | 53.75 | BisGMA 19, HEMA 10 | CQ 0.25, EDMAB 1 | 13.97 (2.89), 24 hrs in water, n=8 |
| 10 | 10 | 53.75 | BisGMA 15, HEMA 10 | CQ 0.25, EDMAB 1 | 14.85 (2.40), 2 hrs in water, n=9 |
| 30 | 10 | 28 | BisGMA 20, HEMA 10 | CQ 0.4, EDMAB 1.6 | 9.42 (1.19), 2 hrs in water, n=8 |
| 3 | 3 | 73 | BisGMA 20, HEMA 10 | CQ 0.25, EDMAB 1 | 15.59 (1.71), 2 hrs in water, n=8 |
| 3 | 58 | 6 | BisGMA 20, HEMA 10 | CQ 0.54, EDMAB 2.2 | 14.99 (2.03), 2 hrs in water, n=8 |
| 3 | 80 | 6 | HEMA 8 | CQ 0.54, EDMAB 2.2 | 12.65 (1.51), 2 hrs in water, n=8 |
| 6.3 | 20.4 | 10 | BisGMA 40.5, HEMA 20.4 | CQ 0.6, EDMAB 1.8 | 17.15 (2.77), 2 hrs in water, n=10, |
| 3 | 10 | 61 | BisGMA 20, HEMA 5 | CQ 0.25, EDMAB 1 | 16.27 (2.33), 2 hrs in water, n=8 |
| 0.6 | 2 | 91 | BisGMA 4, HEMA 2 | CQ 0.05, EDMAB 0.2 | 11.12 (0.6), 2 hrs in water, n=6 |
| 6.3 | 20.4 | 10 | BisGMA 40.5, HEMA 20.4 | CQ 0.6, EDMAB 1.8 | 17.15 (2.77), 2 hrs in water, n=10 |
| 1.4 | 4.5 | 80 | BisGMA9, HEMA 4.52 | CQ 0.11, EDMAB 0.45 | 14.88 (1.60), 2 hrs in water, n=8 |
| 3 | 10 | Acetone 55.75 | BisGMA 20, HEMA 10 | CQ 0.25, EDMAB 1 | 17.06 (2.20), 2 hrs in water, n=8 |
| 3 | 10 | EtOH 44, water 11 | BisGMA 20, HEMA 10 | CQ 0.25, EDMAB 1 | 16.20 (2.04) 2 hrs in water, n=8 |
| 1.4 | 4.5 (BPDM, no DSDM) | 80 | BisGMA 9, HEMA 4.5 | CQ 0.11, EDMAB 0.45 | 15.12 (3.97), 24 hrs, n=5 |

### II. Adhesive Compositions Utilizing Various Comonomers

According to at least one exemplary embodiment, a dental adhesive composition was prepared utilizing the components and concentrations as set forth in Table 2. Similar to those exemplary embodiments set forth in Table 1, for the following exemplary embodiments, absolute ethyl alcohol ("EtOH") was used as the solvent unless otherwise noted; 10-methacryloyloxydecyl dihydrogenphosphates CH₂=C(CH₃)COO(CH₂)₁₀OP(O)(OH)₂ ("MDP") was used as a first acidic acid monomer, DSDM was used as a second acidic acid monomer. However, the comonomers were varied as noted in Table 2 as provided, with the resultant standard shear bond strength results listed. Each noted adhesive composition was mixed in a closed container by shaking the components until all monomers dissolved (approximately four to eight hours). In each of the shear bond strength tests performed in Table 2, Choice 2™ cement was utilized (available from Bisco Inc.) as the cement.

**TABLE 2**

| SBS on Zirconia using Adhesive Compositions (SSBS, in MPa, #5 gel-cap method) | | | | | |
|---|---|---|---|---|---|
| MDP wt. % | DSDM wt. % | EtOH wt. % | Methacrylate wt. % | Initiator wt. % | SSBS in MPa (SD) (stored in water for 24hrs or 2hrs) |
| 3 | 3 | 56 | BisGMA 22, TEGDMA 14 | CQ 0.2, EDMAB 0.6 | 9.60 (2.16), 24 hrs, n=6 |
| 3 | 10 | 52 | BisGMA 21, TEGDMA 13 | CQ 0.2, EDMAB 0.6 | 12.01 (3.44), 24 hrs, n=8 |
| 3 | 15 | 50 | BisGMA 20, TEGDMA 12 | CQ 0.2, EDMAB 0.5 | 11.87 (1.91), 24 hrs, n=8 |
| 5 | 3 | 61 | BisGMA 20, TEGDMA 10 | CQ 0.25 EDMAB 1 | 12.93 (1.64), 2 hrs, n=7 |
| 10 | 10 | 54 | BisGMA 15, TEGDMA 10 | CQ 0.25, EDMAB 1 | 13.85 (3.04), 24 hrs, n=8 |
| 3 | 10 | 66 | BisGMA 20 | CQ 0.25, EDMAB 1 | 16.56 (1.57), 2 hrs, n=8 |
| 3 | 10 | 87 | / | / | 12.53 (1.19), 2 hrs, n=4 |
| 3 | 10 | 53 | BisGMA 20, HEMA 10, MPTS 3 | CQ 0.25, EDMAB 1 | 13.53 (2.53), 24 hrs, n=8 |
| 3 | 10 | 55 | BisGMA 20, HEMA 10, MPTS 1 | CQ 0.25, EDMAB 1 | 16.38 (2.47), 24 hrs, n=11 |
| 5 | 10 | 54 | BisGMA 20, HEMA 10, MPTS 1 | CQ 0.25, EDMAB 1 | 14.96 (3.57), 24 hrs, n=8 |

It will be appreciated that initial bond strengths as shown are high throughout a range of examples, including those examples having a high percentage of the first and second acidic monomers. However, accelerated aging tests of the adhesive compositions at high temperature indicate that those compositions with a high percentage of the first and second acidic monomers form a high build surface on the zirconia surface and may weaken significantly over time when exposed to high temperatures in the presence of water. On the contrary, those adhesive compositions utilizing less than 20% monomers, including those utilizing less than 10-15% acidic monomers show high initial strength and maintain long term strength within clinically acceptable ranges, for example, displaying long term SSBS of 10 MPa or better when measured with the #5 gel cap method after 72 hours in 100° water.

### III. Adhesive Compositions Compared

In one exemplary embodiment, a dental adhesive composition was prepared utilizing 55.75 % by wt. ethanol, 3 % by wt. MDP, 10 % by wt. DSDM, 20 % by wt. BisGMA, 10 % by wt. HEM A, 0.25 % by wt. CQ, and 1 % by wt. EDMAB, and the shear bond strength of zirconia materials treated with the dental adhesive composition to various cements was tested against commercially available products marketed as improving the bonding strength to zirconia materials. In particular, the commercially available adhesive or primer system for zirconia was applied to the zirconia surface, and was allowed to cure per the directions. Those adhesive or primer systems include: Clearfil Ceramic Primer (available from Kuraray); Metal/Zirconia Primer (available from Ivoclar Vivadent); and the composition discussed above, and referred to below as "Zirconia Adhesive A." The cements listed in Table 3 below were then utilized to bond the primed zirconia surface and tested as discussed above. Those results are listed in Table 3 below. It will be appreciated that the SBS displayed when utilizing Adhesive A were significantly higher than any other commercially available product, with the highest test results often resulting in two to three times the shear bond strength of other commercially available zirconia primers or adhesives.

**TABLE 3.**

| SBS on Zirconia by using an Adhesive composition as described above and commercial zirconia bonding products | | |
|---|---|---|
| **Primer or Adhesive** | **Cement** | **SSBS in MPa (SD)** |
| Clearfil Ceramic Primer (Kuraray) | Panavia F2.0 (Kuraray) | **7.18** (0.83), 2 hrs, n=8 |
| Clearfil Ceramic Primer (Kuraray) | Panavia F2.0 (Kuraray) | **7.48** (0.89), 24 hrs, n=10 |
| Clearfil Ceramic Primer (Kuraray) | Duolink (Bisco) | **9.34** (1.91), 24 hrs, n=9 |
| Clearfil Ceramic Primer (Kuraray) | Choice 2 (Bisco) | **8.16** (2.78), 24 hrs, n=9 |
| Metal/Zirconia Primer (Ivoclar Vivadent) | Choice 2 (Bisco) | **5.71** (3.22), 24 hrs, n=10 |
| Metal/Zirconia Primer (Ivoclar Vivadent) | Duolink (Bisco) | **3.84** (2.57), 24 hrs, n=9 |
| Metal/Zirconia Primer (Ivoclar Vivadent) | MultilinkAutomix (Ivoclar Vivadent) | **5.19** (1.88), 2 hrs, n=8 |
| Metal/Zirconia Primer (Ivoclar Vivadent) | MultilinkAutomix (Ivoclar Vivadent) | **2.73** (2.36), 24 hrs, n=10 |
| Zirconia Adhesive A | Panavia F2.0 (Kuraray) | **17.22** (1.98), 24 hrs, n=9 |
| Zirconia Adhesive A | MultilinkAutomix (Ivoclar Vivadent) | **15.56** (3.58), 24 hrs, n=9 |
| Zirconia Adhesive A | Duolink (Bisco) | **14.97** (1.47), 2 hrs, n=9 |
| Zirconia Adhesive A | Duolink (Bisco) | **16.52** (1.17), 24 hrs, n=10 |
| Zirconia Adhesive A | Choice 2 (Bisco) | **15.69** (2.05), 2 hrs, n=10 |
| Zirconia Adhesive A | Choice 2 (Bisco) | **16.63** (2.48), 24 hrs, n-14 |

### IV. Adhesive Composition Shear Bond Strength When Used on Substrates other than Zirconia

In one exemplary embodiment, a dental adhesive composition was prepared utilizing 55.75 % by wt. ethanol, 3 % by wt. MDP, 10 % by wt. DSDM, 20 % by wt. BisGMA, 10 % by wt. HEMA, 0.25 % by wt. CQ, and 1 % by wt. EDMAB, and the shear bond strength of the dental adhesive composition was tested for various substrates other than zirconia, indicating the increased bonding strength shown when the dental adhesive composition is used as described in Example II above. In each of the shear bond strength tests performed in Table 4, Choice 2™ cement was utilized (available from Bisco Inc.) as the cement. These shear bond strengths were compared to bonding tests where only the Choice 2™ cement was utilized. It will be appreciated from the results of Table 4 that use of the dental adhesive composition on the substrate prior to application of the cement improved shear bond strength for all substrates.

**TABLE 4.**

| SBS on various dental substrates by using above-described Adhesive composition | | |
|---|---|---|
| Substrate | SSBS in MPa (SD), #5-gel cap Above Described Adhesive | SSBS in MPa (SD), #5-gel cap No Adhesive |
| Zirconia | **15.69** (2.05), n=10 | **1.46** (0.29), n=2 |
| Titanium | **15.07** (2.46), n=8 | **7.32** (1.12), n=8 |
| Stainless steel | **15.15** (2.74), n=9 | **7.19** (0.65), n=6 |
| gold | **8.71** (1.67), n=9 | **5.54** (2.21), n=7 |
| RexIII | **15.07** (2.14), n=10 | **6.14** (0.44), n=8 |
| Dentin - unetched | **14.81** (2.50), n=10, | |
| Dentin - etched | **23.47** (5.09, n=9 | |

Although the invention has been described in detail with reference to preferred embodiments, variations and modifications exist within the scope of the invention as described and defined in the following claims.

## Claims

1. A dental restoration system comprising:
a zirconia-based dental appliance; and
an adhesive composition applied to the surface of the zirconia-based dental appliance, comprising:
a. a first acidic monomer having an ethylenically unsaturated polymerizable group of the general formula CH₂=CX-C(O)-R wherein X is hydrogen, methyl or a lower alkyl group, and wherein R comprises a phosphoric acid group or phosphonic acid group having the general formula -OP(O)(OH)₂, -OP(O)(OH), -C-P(O)(OH)₂ or -C-P(O)(OH);
b. a second acidic monomer having an ethylenically unsaturated polymerizable group of the general formula CH₂=CX-C(O)-R₁ wherein X is hydrogen, methyl or a lower alkyl group, and wherein R₁ comprises a carboxylic acid group;
c. wherein the first acidic monomer comprises 1% to 5% of the adhesive composition by weight; and
d. wherein the second acidic monomer comprises 2% to 10% of the adhesive composition by weight.

2. The dental restoration system of claim 1, wherein the first acidic monomer is selected from the group consisting of CH₂=C(CH₃)COO(CH₂)ₙOP(O)(OH)₂ and CH₂=C(CH₃)COO(CH₂)ₙP(O)(OH)₂, wherein n is an integer from 2 to 20.

3. The dental restoration system of claim 1, wherein the second acidic monomer is selected from the group consisting of: 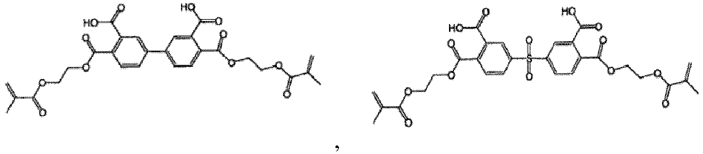 and

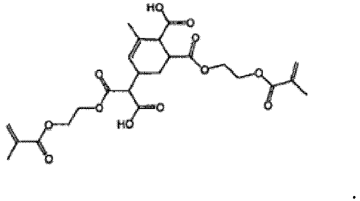

4. The dental restoration system of claim 1, further comprising at least one comonomer selected from the group consisting of 2-hydroxyethyl methacrylate, bisphenol A diglycidyl methacrylate, triethylene glycol dimethacrylate, 1,6-hexanediol dimethacrylate, ethoxylated bisphenol A diglycidyl methacrylate, and urethane dimethacrylate, preferably further comprising an initiator, wherein the initiator may comprise at least one photosensitive ketone and at least one tertiary amine.

5. The dental restoration system of claim 1, wherein neither of the acidic monomers contain a halogen group.

6. The dental restoration system of claim 1, wherein the ratio of the first acidic monomer to the second acidic monomer is between 1:4 and 2:1, respectively.

7. The dental restoration system of claim 4, wherein the at least one comonomer comprises two comonomers selected from the group consisting of 2-hydroxyethyl methacrylate, bisphenol A diglycidyl methacrylate, triethylene glycol dimethacrylate, 1,6-hexanediol dimethacrylate, ethoxylated bisphenol A diglycidyl methacrylate, and urethane dimethacrylate.

8. The dental restoration system of claim 4, wherein the initiator comprises 0.05% to 2% of the adhesive composition by weight.

9. The dental restoration system of claim 7, wherein the adhesive system displays an initial bond strength to zirconia that is greater than 10 MPa as measured with the #5 gel cap method when polymerized or, wherein the adhesive displays an initial bond strength to zirconia that is at least 14 MPa as measured with the #5 gel cap method when polymerized.

10. An extra-oral method of bonding a zirconia-based dental appliance to a surface, comprising:
a. providing a first acidic monomer having an ethylenically unsaturated polymerizable group of the general formula CH₂=CX-C(O)-R wherein X is hydrogen, methyl or a lower alkyl group, and wherein R is a phosphoric acid group or phosphonic acid group having the general formula -OP(O)(OH)₂, - OP(O)(OH), -C-P(O)(OH)₂ or -C-P(O)(OH), the first acidic monomer comprising 1% to 5% of the provided materials by weight;
b. providing a second acidic monomer having an ethylenically unsaturated polymerizable group of the general formula CH₂=CX-C(O)-R₁ wherein X is hydrogen, methyl or a lower alkyl group, and wherein R₁ comprises a carboxylic acid group, the second acidic monomer comprising 2% to 10% of the provided materials by weight;
c. providing at least one comonomer selected from the group consisting of 2-hydroxyethyl methacrylate, bisphenol A diglycidyl methacrylate, triethylene glycol dimethacrylate, 1,6-hexanediol dimethacrylate, ethoxylated bisphenol A diglycidyl methacrylate, and urethane dimethacrylate, the comonomer comprising 4% to 36% of the provided materials by weight;
d. providing an initiator comprising at least one photosensitive ketone and at least one tertiary amine, the initiator comprising 0.05% to 3% of the provided materials by weight;
e. providing a solvent comprising 40% to 90% of the provided materials by weight;
f. combining all provided materials and applying those materials to a zirconia-based dental appliance;
g. polymerizing the combined materials applied to the zirconia dental appliance wherein the initial bond between the polymerized materials and the zirconia is at least about 10 MPa as measured with the #5 gel cap method.

11. The method of claim 10, wherein none of the provided monomers comprise a halogen group.

12. The method of claim 10, wherein the initial bond between the polymerized materials and the zirconia is at least about 14 MPa as measured with the #5 gel cap method or wherein the bond between the polymerized materials and the zirconia is at least about 10 MPa as measured with the #5 gel cap method when exposed to water at 100° for 72 hours.

13. The method of claim 10, wherein the provided materials are polymerized via light curing.

## Patentansprüche

1. Ein Zahnrestaurationssystem, umfassend
ein Zirkoniumdioxid-basiertes Dentalgerät; und
eine auf das Zirkoniumdioxid-Dentalgerät aufgetragene Dentalklebstoffzusammensetzung, umfassend:
a. ein erstes saures Monomer mit einer ethylenisch ungesättigten polymerisierbaren Gruppe der allgemeinen Formel CH2=CX-C(O)-R, worin X Wasserstoff, Methyl oder eine Niederalkylgruppe ist und worin R eine Phosphorsäuregruppe oder Phosphonsäuregruppe mit der allgemeinen Formel - OP(O)(OH)₂, -OP(O)(OH), -C-P(O)(OH)₂ und -C-P(O)(OH) ist;
b. ein zweites saures Monomer mit einer ethylenisch ungesättigten polymerisierbaren Gruppe der allgemeinen Formel CH2=CX-C(O)-R₁, worin X Wasserstoff, Methyl oder eine Niederalkylgruppe ist und worin R₁ eine Carbonsäuregruppe ist;
c. wobei das erste saure Monomer 1 bis 5 Gew.-% des Dentalklebstoffsystems ausmacht; und
d. worin das zweite saure Monomer 2 bis 10 Gew.-% des Dentalklebstoffsystems ausmacht.

2. Das Zahnrestaurationssystem nach Anspruch 1, wobei das erste saure Monomer ausgewählt ist aus der Gruppe bestehend aus CH₂=C(CH₃)COO(CH₂)nOP(O)(OH)₂ und CH₂=C(CH₃)COO(CH₂)nP(O)(OH)₂, worin n eine ganze Zahl von 2 bis 20 ist.

3. Das Zahnrestaurationssystem nach Anspruch 1, wobei das zweite saure Monomer ausgewählt ist aus der Gruppe bestehend aus 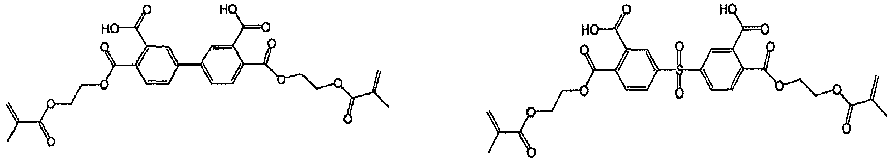 und

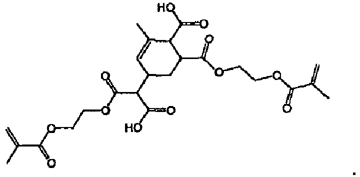

4. Das Zahnrestaurationssystem nach Anspruch 1, weiterhin umfassend mindestens ein Comonomer, ausgewählt aus der Gruppe bestehend aus 2-Hydroxyethylmethacrylat, Bisphenol A-Diglycidylmethacrylat, Triethylenglykol-Dimethacrylat, 1,6-Hexandioldimethacrylat, ethoxyliertem Bisphenol A-Diglycidylmethacrylat und Urethandimethacrylat; bevorzugt weiterhin umfassend einen Initiator, wobei der Initiator mindestens ein lichtempfindliches Keton und mindestens ein tertiäres Amin umfassen kann.

5. Das Zahnrestaurationssystem nach Anspruch 1, wobei keins der sauren Monomere eine Halogen-Gruppe enthält.

6. Das Zahnrestaurationssystem nach Anspruch 1, wobei das Verhältnis des ersten sauren Monomers zum zweiten sauren Monomer zwischen 1:4 und 2:1 liegt.

7. Das Zahnrestaurationssystem nach Anspruch 4, wobei der wenigstens eine Comonomer, zwei Comonomere ausgewählt aus der Gruppe bestehend aus 2-Hydroxyethylmethacrylat, Bisphenol A-Diglycidylmethacrylat, Triethylenglykol-Dimethacrylat, 1,6-Hexandioldimethacrylat, ethoxyliertem Bisphenol A-Diglycidylmethacrylat und Urethandimethacrylat umfasst.

8. Das Zahnrestaurationssystem nach Anspruch 4, wobei der Initiator 0,05 bis 2 Gew.-% der Klebstoffzusammensetzung ausmacht.

9. Das Zahnrestaurationssystem nach Anspruch 7, wobei das Klebesystem eine anfängliche Haftfestigkeit auf Zirkoniumdioxid zeigt, die größer als 10 MPa ist, gemessen mit der #5 Gel-Cap-Methode nach Polymerisation oder wobei das Klebesystem eine anfängliche Haftfestigkeit auf Zirkoniumdioxid zeigt, die größer als 14 MPa ist, gemessen mit der #5 Gel-Cap-Methode nach Polymerisation.

10. Verfahren zum Binden von Zirkoniumdioxid an eine Oberfläche, umfassend:
a. Bereitstellen eines ersten sauren Monomers mit einer ethylenisch ungesättigten polymerisierbaren Gruppe der allgemeinen Formel CH2=CX-C(O)-R, worin X Wasserstoff, Methyl oder eine Niederalkylgruppe ist und worin R eine Phosphorsäuregruppe oder Phosphonsäuregruppe mit der allgemeinen Formel - OP(O)(OH)₂, -OP(O)(OH), -C-P(O)(OH)₂ und -C-P(O)(OH) ist, wobei das erste saure Monomer 1 bis 5 Gew.-% der bereitgestellten Materialien ausmacht;
b. Bereitstellen eines zweiten sauren Monomers mit einer ethylenisch ungesättigten polymerisierbaren Gruppe der allgemeinen Formel CH₂=CX-C(O)-R₁ worin X Wasserstoff, Methyl oder eine Niederalkylgruppe ist und worin R₁ eine Carbonsäuregruppe umfasst, wobei das zweite saure Monomer 2 bis 10 Gew.-% der bereitgestellten Materialien ausmacht;
c. Bereitstellen mindestens eines Comonomers, ausgewählt aus der Gruppe bestehend aus 2-Hydroxyethylmethacrylat, Bisphenol A-Diglycidylmethacrylat, Triethylenglykol-Dimethacrylat, 1,6-Hexandioldimethacrylat, ethoxyliertem Bisphenol A-Diglycidylmethacrylat und Urethandimethacrylat, wobei das Comonomer 4 bis 36 Gew.-% der bereitgestellten Materialien ausmacht;
d. Bereitstellen eines Initiators, umfassend mindestens ein lichtempfindliches Keton und mindestens ein tertiäres Amin wobei der Initiator 0,05 bis 3 Gew.-% der bereitgestellten Materialien ausmacht;
e. Bereitstellen eines Lösungsmittels, dass 40 bis 90 Gew.-% der bereitgestellten Materialien ausmacht;
f. Kombinieren aller bereitgestellten Materialien und Aufbringen dieser Materialien auf ein Zirkoniumdioxid-Dentalgerät; und
g. Polymerisieren der kombinierten Materialien, die auf das Zirkoniumdioxid-Dentalgerät aufgebracht werden, wobei die anfängliche Bindung zwischen den polymerisierten Materialien und dem Zirkoniumdioxid mindestens etwa 10 MPa beträgt, gemessen mit der #5-Gel-Cap-Methode.

11. Das Verfahren nach Anspruch 10, wobei keins der bereitgestellten Monomere eine Halogen-Gruppe enthält.

12. Verfahren nach Anspruch 10, wobei die anfängliche Bindung zwischen den polymerisierten Materialien und dem Zirkoniumdioxid mindestens 14 MPa beträgt, gemessen mit der #5-Gel-Cap-Methode oder wobei die Bindung zwischen den polymerisierten Materialien und dem Zirkoniumdioxid mindestens10 MPa beträgt, gemessen mit der #5 Gel-Cap-Methode, wenn sie 72 Stunden lang bei 100° C Wasser ausgesetzt wird.

13. Das Verfahren nach Anspruch 10, wobei die bereitgestellten Materialien mittels Lichthärtung polymerisiert werden.

## Revendications

1. Système de restauration dentaire comprenant :
un appareil dentaire à base de zircone, et
une composition adhésive appliquée sur la surface de l'appareil dentaire à base de zircone comprenant :
a. un premier monomère acide ayant un groupe polymérisable à insaturation éthylénique de formule générale CH₂=CX-C(O)-R dans laquelle X représente un hydrogène, un groupe méthyl ou un groupe alkyl inférieur et R renferme un groupe acide phosphorique ou un groupe acide phosphonique ayant la formule générale -OP(O)(OH)₂, - OP(O)(OH),-C-P(O)(OH)₂ ou -C-P(O)(OH),
b. un second monomère acide ayant un groupe polymérisable à insaturation éthylénique de formule générale CH₂=CX-C(O)-R₁ dans laquelle X représente un hydrogène, un groupe méthyl ou un groupe alkyl inférieur et R₁ renferme un groupe acide carboxylique,
c. le premier monomère acide constituant 1% à 5% en poids de la composition adhésive, et
d. le second monomère acide constituant 2% à 10% en poids de la composition adhésive.

2. Système de restauration dentaire conforme à la revendication 1, dans lequel le premier monomère acide est choisi dans le groupe formé par CH₂=C(CH₃)COO(CH₂)ₙOP(O)(OH)₂ et CH₂=C(CH₃)COO(CH₂)ₙP(O)(OH)₂, n étant un nombre entier compris entre 2 et 20.

3. Système de restauration dentaire conforme à la revendication 1, dans lequel le second monomère acide est choisi dans le groupe formé par : 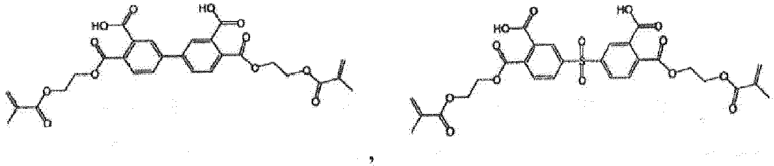 et

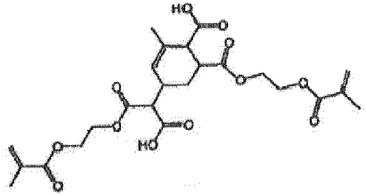

4. Système de restauration dentaire conforme à la revendication 1, renfermant en outre au moins un co-monomère choisi dans le groupe formé par les composés suivants : 2-hydroxyethyl méthacrylate, bisphénol A diglycidyl méthacrylate, tri éthylène glycol di méthacrylate, 1,6-hexanediol di méthacrylate, bisphénol A diglycidyl méthacrylate ethoxyle, et uréthane di méthacrylate, et renfermant de préférence en outre un initiateur, l'initiateur pouvant renfermer au moins une cétone photosensible et au moins une amine tertiaire.

5. Système de restauration dentaire conforme à la revendication 1, dans lequel aucun des monomères acides ne renferme un groupe halogène.

6. Système de restauration dentaire conforme à la revendication 1, dans lequel le rapport respectif du premier monomère acide au second monomère acide est compris entre 1:4 et 2:1.

7. Système de restauration dentaire conforme à la revendication 4, dans lequel le comonomère comporte deux comonomères choisis dans le groupe formé par les composés suivants : 2-hydroxyethyl méthacrylate, bisphénol A diglycidyl méthacrylate, tri éthylène glycol di méthacrylate, 1,6-hexanediol di méthacrylate, bisphénol A diglycidyl méthacrylate ethoxyle, et uréthane di méthacrylate.

8. Système de restauration dentaire conforme à la revendication 4, dans lequel l'initiateur constitue 0,05% à 2% en poids de la composition adhésive.

9. Système de restauration dentaire conforme à la revendication 7, dans lequel le système adhésif présente une force d'adhésion initiale à la zircone qui est supérieure à 10 MPa mesurée par la méthode dite « #5 gel cap » lorsqu'il est polymérisé ou l'adhésif présente une force d'adhésion initiale à la zircone qui est égale à au moins 14 MPa lorsque mesurée par la méthode dite « #5 gel cap » lorsqu'il est polymérisé.

10. Procédé extra-oral de liaison d'une application dentaire à base de zircone à une surface comprenant des étapes consistant à :
a. se procurer un premier monomère acide ayant un groupe polymérisable à insaturation éthylénique de formule générale CH₂=CX-C(O)-R dans laquelle X représente un hydrogène, un groupe méthyl ou un groupe alkyl inférieur et R représente un groupe acide phosphorique ou un groupe acide phosphonique ayant la formule générale -OP(O)(OH)₂,-OP(O)(OH), -C-P(O)(OH)₂ ou -C-P(O)(OH), le premier monomère acide constituant 1% à 5% en poids des matériaux fournis,
b. se procurer un second monomère acide ayant un groupe polymérisable à insaturation éthylénique de formule générale CH₂=CX-C(O)-R₁ dans laquelle X représente un hydrogène, un groupe méthyl ou un groupe alkyl inférieur et R₁ renferme un groupe acide carboxylique, le second monomère acide constituant 2% à 10% en poids des matériaux fournis,
c. se procurer au moins un comonomère choisi dans le groupe formé par les composés suivants 2-hydroxyethyl methacrylate, bisphenol A diglycidyl methacrylate, tri éthylène glycol dimethacrylate, 1,6-hexanediol dimethacrylate, bisphenol A diglycidyl methacrylate ethoxyle, et uréthane dimethacrylate, le comonomère constituant 4% à 36% en poids des matériaux fournis,
d. se procurer un initiateur renfermant au moins une cétone photosensible et au moins une amine tertiaire, l'initiateur constituant 0,05% à 3% en poids des matériaux fournis,
e. se procurer un solvant constituant 40% à 90% en poids des matériaux fournis,
f. combiner tous les matériaux fournis et appliquer ces matériaux sur un appareil dentaire à base de zircone,
g. polymériser les matériaux combinés appliqués sur l'appareil dentaire à base de zircone, la force d'adhésion initiale entre les matériaux polymérisés et la zircone étant égale à au moins environ 10 MPa mesurée par la méthode dite « #5 gel cap ».

11. Procédé conforme à la revendication 10,
selon lequel aucun des monomères fournis ne renferme de groupe halogène.

12. Procédé conforme à la revendication 10,
selon lequel la force d'adhésion initiale entre les matériaux polymérisés et la zircone est d'au moins environ 14 MPa mesurée par la méthode dite « #5 gel cap » ou la force d'adhésion entre les matériaux polymérisés et la zircone est égale à au moins 10 MPa mesurée par la méthode dite « #5 gel cap » après une exposition à l'eau à 100° pendant 72 heures.

13. Procédé conforme à la revendication 10,
selon lequel les matériaux fournis sont polymérisés par réticulation sous l'action de la lumière.
